# EUROPEAN PATENT SPECIFICATION

(11) **EP 2 178 512 B1**
(45) Date of publication and mention of the grant of the patent: **09.03.2011**
(21) Application number: 08771565.2
(22) Date of filing: 20.06.2008
(51) Int. Cl.: A61K 9/20, C07D 213/42, A61K 31/395, A61K 45/06

(54) **TABLETED COMPOSITIONS CONTAINING ATAZANAVIR**
TABLETTIERTE ATAZANAVIRHALTIGE ZUSAMMENSETZUNGEN
COMPOSITIONS EN COMPRIMÉS CONTENANT DE L'ATAZANAVIR

(30) Priority: 22.06.2007 US 945691 P
(43) Date of publication of application: 28.04.2010
(73) Proprietor: Bristol-Myers Squibb Company, Princeton, NJ 05843-4000 (US)
(72) Inventor: KOO, Otilia May Yue, New Brunswick, New Jersey 08903 (US); NIKFAR, Faranak, New Brunswick, New Jersey 08903 (US); DIAZ, Steven, Martinsville, New Jersey 08836 (US)
(74) Representative: Reitstötter - Kinzebach
(86) International application number: PCT/US2008/067629
(87) International publication number: WO 2009/002826

(56) References cited:
- US-A- 5 849 911
- US-A1- 2005 256 202
- WALMSLEY SHARON: "Protease inhibitor-based regimens for HIV therapy: safety and efficacy." JOURNAL OF ACQUIRED IMMUNE DEFICIENCY SYNDROMES (1999) 1 JUN 2007, vol. 45 Suppl 1, 1 June 2007 (2007-06-01), pages S5-13 ; quiz, XP002545190 ISSN: 1525-4135

## Description

### FIELD OF THE INVENTION

The present invention relates to pharmaceutical compositions, processes, and treatment methods

### BACKGROUND OF THE INVENTION

Human immunodeficiency virus (HIV) has been identified as the etiological agent responsible for acquired immune deficiency syndrome (AIDS), a serious disease characterized by destruction of the immune system and the inability to fight off life threatening opportunistic infections.

U.S. Patent No. 5,849,911 to Fässler et al. discloses a series of azapeptide HIV protease inhibitors (which includes atazanavir) which have the structure 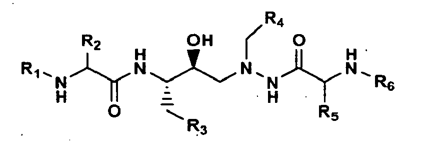 wherein
R₁ is lower alkoxycarbonyl,
R₂ is secondary or tertiary lower alkyl or lower alkylthio-lower alkyl,
R₃ is phenyl that is unsubstituted or substituted by one or more lower alkoxy radicals, or C₄-C₈ cycloalkyl,
R₄ is phenyl or cyclohexyl each substituted in the 4-position by unsaturated heterocyclyl that is bonded by way of a ring carbon atom, has from 5 to 8 ring atoms, contains from 1 to 4 hetero atoms selected from nitrogen, oxygen, sulfur, sulfinyl (-SO-) and sulfonyl (-SO₂-) and is unsubstituted or substituted by lower alkyl or by phenyl-lower alkyl,
R₅, independently of R₂, has one of the meanings mentioned for R₂, and
R₆, independently of R₁, is lower alkoxycarbonyl, or a salt thereof, provided that at least one salt-forming group is present which includes various pharmaceutically acceptable acid addition salts thereof.

U.S. Patent No. 6,087,383 to Singh et al. discloses the bisulfate salt of the azapeptide HIV protease inhibitor known as atazanavir which has the structure 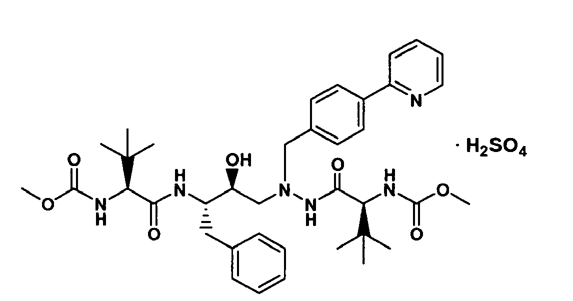 (referred to herein as "atazanavir bisulfate" or "atazanavir sulfate").

U.S. Patent Publication No. US20050256202A1, published November 17, 2005, discloses processes for preparing the HIV protease inhibitor atazanavir bisulfate and novel forms thereof.

Atazanavir is commercially available as a prescription medicine from Bristol-Myers Squibb Company, New York, under the tradename REYATAZ^{®} (atazanavir sulfate) for the treatment of HIV. Approved in 2003 by the U.S. Food and Drug Administration, REYATAZ^{®} (atazanavir sulfate) is currently available in the form of 100 milligram ("mg"), 150 mg, 200 mg, and 300 mg capsules. Patient demand for REYATAZ^{®}) (atazanavir sulfate) has been substantial and continues to grow.

The combination of atazanavir and abacavir in HIV therapy is disclosed in: Protease Inhibitor-Based Regimes for HIV-therapy. S. Walmsley. J. Acquir. Immune Defic. Syndr. 2007, 45(supplement 1):S5-S13.

Currently, atazanavir sulfate is not commercially available in a tablet form. Although delivery of medicines in capsule form is often desired, delivery in tablet form can be advantageous. For example, tablets can provide: reduced liability to tampering; ease of swallowing; easily dividable doses; and the ability to combine drugs in fixed dose combination in single layer or multi-layer tablets, e.g., bilayer tablets.

### SUMMARY OF THE INVENTION

The invention encompasses compressed tablets containing compressed tablets comprise atazanavir sulfate, optionally with another active agents, e.g., anti-HIV agents. The abacavir sulfate and granules that contain atazanavir sulfate and an intragranular lubricant that can be used to make the tablets, compositions comprising a plurality of the granules, processes for making the granules and tablets, and methods of treating HIV.

By the present invention, it is now possible to provide atazanavir tablets in a tableted form. In accordance with the present invention, a lubricant is combined with atazanavir sulfate during the preparation of the granules. Quite surpisingly, the tablets formed from the granules can have desireable tablet dissolution properties and desireable processing properties during manufacture.

### DETAILED DESCRIPTION OF THE INVENTION

In accordance with the present invention, the method in which the atazanavir sulfate is prepared is not critical. Typically, the atazanavir sulfate is present as Form A, Form E3 or Pattern C, preferably in particular in pharmaceutically acceptable form. Often, the crystalline forms of atazanavir and salts thereof are in substantially pure form. These forms are described in U.S. Patent Publication No. US20050256202A1, published November 17, 2005. The term "pharmaceutically acceptable", as used herein, refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio. The term "substantially pure" means a compound having a chemical purity of at least about 90 wt%, preferably at least about 95 wt%, more preferably at least about 98 wt% of the compound and less than about 10 wt%, preferably less than about 5 wt%, and more preferably less than about 2 wt% of other compounds having a different chemical structure than the compound.

In one suitable method, atazanavir in the form of its free base may be prepared by treating a solution of a protected triamine salt of the structure 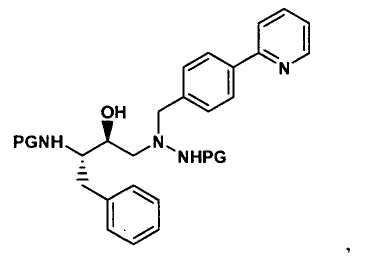 (where PG represents a protecting group such as t-butyloxycarbonyl (Boc) or trifluoroacetyl, preferably Boc, with an acid, preferably hydrochloric acid (where Boc is used), or a base (where trifluoroacetyl is used) in the presence of an organic solvent such as methylene chloride, tetrahydrofuran, or methanol, which solvent is preferably methylene chloride, at a temperature within the range from about 25 to about 50°C, preferably from about 30 to about 40°C, to form the triamine acid salt, preferably the hydrogen chloride salt of the structure 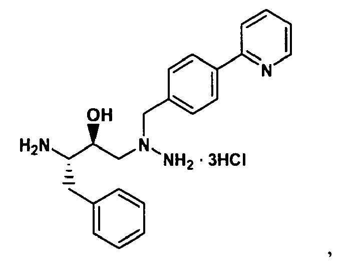 and without isolating the triamine acid salt, reacting the triamine acid salt with an active ester of an acid of the structure 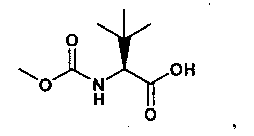 preferably the active ester of the structure 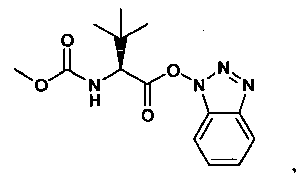 in the presence of a base such as K₂HPO₄, diisopropylethylamine, N-methylmorpholine, sodium carbonate, or potassium carbonate, preferably K₂HPO₄ in the presence of an organic solvent such as methylene chloride, a mixture of ethyl acetate and butyl acetate, acetonitrile or ethyl acetate, preferably methylene chloride, at a temperature within the range from about 25 to about 50°C, preferably from about 30 to about 40°C to form atazanavir free base.

The protected triamine starting material may be prepared by reacting the epoxide 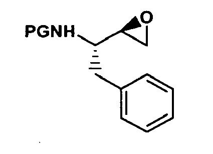 where PG is preferably Boc such as N-(tert-butyloxycarbonyl)-2(S)-amino- 1-phenyl-3(R)-3,4-epoxy-butane, with the hydrazine carbamate 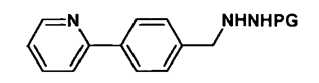 where PG is preferably Boc in the presence of isopropyl alcohol or other alcohol such as ethanol or butanol.

One suitable method for preparing Form A crystals of atazanavir sulfate salt, a modified cubic crystallization technique is employed wherein atazanavir free base is dissolved in an organic solvent in which the atazanavir sulfate salt is substantially insoluble and includes acetone, a mixture of acetone and N-methyl pyrrolidone, ethanol, a mixture of ethanol and acetone and the like, to provide a solution having a concentration of atazanavir free base within the range from about 6.5 to about 9.7% by weight, preferably from about 6.9 to about 8.1% by weight atazanavir free base.

The solution of atazanavir free base is heated at a temperature within the range from about 35 to about 55°C, preferably from about 40 to about 50°C, and reacted with an amount of concentrated sulfuric acid (containing from about 95 to about 100% H₂SO₄) to react with less than about 15%, preferably from about 5 to less than about 12%, more preferably from about 8 to about 10% by weight of the total atazanavir free base. Thus, the starting solution of atazanavir free base will be initially reacted with less than about 15%, preferably from about 5 to about 12%, by weight of the total amount of sulfuric acid to be employed. During the reaction, the reaction mixture is maintained at a temperature within the range from about 35 to about 55°C, preferably from about 40 to about 50°C.

The reaction is allowed to continue for a period from about 12 to about 60 minutes, preferably from about 15 to about 30 minutes.

The reaction mixture is seeded with crystals of Form A atazanavir sulfate employing an amount of seeds within the range from about 0.1 to about 80% by weight, preferably from about 3 to about 8% by weight, based on the weight of atazanavir free base remaining in the reaction mixture while maintaining the reaction mixture at a temperature within the range from about 35 to about 55°C, preferably from about 40 to about 50°C.

The reaction is allowed to continue until crystallization begins. Thereafter, sulfuric acid is added in multiple stages at an increasing rate according to the cubic equation as described in U.S. Patent Publication No. US20050256202A1, published November 17, 2005 to form atazanavir sulfate which upon drying produces Form A crystals.

The crystal particle size and morphology of the atazanavir sulfate salt formed are dependent on the addition rate of the sulfuric acid, which determines the crystallization rate. It has been found that a modified "cubic" crystallization technique (acid added at an increasing rate according to a cubic equation) provides relatively larger, more well defined atazanavir sulfate crystals, along with a narrower particle size range and fewer fines, than a constant addition rate crystallization. The slow initial acid flow rate has been shown to favor crystal growth over secondary nucleation. Thus, as the surface area increases with particle size, the seed bed is able to accept the increasing acid flow rate without inducing secondary nucleation. The slow initial addition rate allows time for the crystals to grow larger, increasing the mean size. The cubic crystallization provides a less compressible filter cake, which aids in effective cake deliquoring and washing, as well as giving a more easily dried product with fewer hard lumps than the constant addition rate crystallized product.

Pattern C material may be prepared, for example, by exposing Form A crystals to water followed by drying. Pattern C material may also be formed by exposing crystals of Form A to high relative humidity of greater than about 95% RH, preferably from about 95 to about 100% RH (water vapor), for at least 24 hours, preferably from about 24 to about 48 hours. Pattern C material may also be prepared by wet granulating atazanavir sulfate Form A to produce granules of atazanavir sulfate and then drying the granules.

The Form E3 may be prepared, for example, by slurrying atazanavir free base in ethanol, treating the slurry with concentrated sulfuric acid employing a molar ratio of acid: free base with the range from about 1:1 to about 1.1:1, heating the resulting solution at from about 30 to about 40°C, seeding the solution with ethanol wet E3 crystals of atazanavir sulfate, treating the mixture with heptane (or other solvent such as hexane or toluene), filtering, and drying to yield atazanavir sulfate Form E3 (triethanol solvate). The seeding step will employ an amount of seeds to effect formation of E3 crystals, for example a molar ratio of atazanavir sulfate E-3 seeds:free base within the range from about 0.02:1 to about 0.04:1.

Further details concerning the preparation of the atazanavir sulfate suitable for use in accordance with the present invention are described in U.S. Patent Publication No. US20050256202A1, published November 17, 2005.

The present invention contemplates the use of any pharmaceutically acceptable ingredients, such as, for example, lubricants, disintegrants, binders, fillers (also referred to as "compression aids"), surfactants, film coatings, and solvents. Examples of some of these ingredients are set forth below and are described in more detail in the Handbook of Pharmaceutical Excipients, Second Edition, Ed. A. Wade and P. J. Weller, 1994, The Pharmaceutical Press, London, England. The selection and amounts of such ingredients to be used in accordance with the present invention are not critical and can be determined by one skilled in the art.

Examples of lubricants suitable for use in accordance with the invention, but are not limited to, magnesium stearate, zinc stearate, calcium stearate, stearic acid, palmitic acid, sodium stearyl fumarate, sodium benzoate, sodium lauryl sulfate, glyceryl monostearate, glyceryl palmitostearate; hydrogenated castor oil, hydrogenated vegetable oil, mineral oil, carnauba wax, and polyethylene glycol. In accordance with the invention, ingredients also referred to as "glidants" are intended to be included within the scope of lubricants. Examples include, but are not limited to, silicon dioxide, calcium silicate, calcium phosphate and talc.

Examples of disintegrants suitable for use in accordance with the invention, but are not limited to, croscarmellose sodium, crospovidone, potato starch, pregelatinized starch, corn starch, sodium starch glycolate, microcrystalline cellulose, powdered cellulose, methylcellulose, carboxymethylcellulose calcium, carboxymethylcellulose sodium, alginic acid, colloidal silicon dioxide, guar gum, Magnesium aluminum silicate, polyacrilin potassium and sodium alginate.

Examples of binders suitable for use in accordance with the invention, but are not limited to, acacia, carbomer, dextrin, gelatin, guar gum, hydrogenated vegetable oil, methylcellulose, ethyl cellulose, cellulose acetate, hydroxyethyl cellulose, hydroxypropyl cellulose, hydroxypropyl methylcellulose, carboxymethylcellulose sodium, glucose, lactose, magnesium aluminaum silicate, maltodextrin, polymethacrylates, povidone, polyvinyl pyrrolidone, corn starch, pregelatinized starch, alginic acid, sodium alginate, zein, carnauba wax, paraffin, spermaceti, polyethylenes and microcrystalline wax.

Examples of fillers suitable for use in accordance with the invention, but are not limited to, microcrystalline cellulose, lactose, sucrose, starch, pregelatinized starch, dextrose, dextrates, dextrin, mannitol, fructose, xylitol, sorbitol, corn starch, modified corn starch, inorganic salts such as calcium carbonate, magnesium carbonate, magnesium oxide, calcium phosphate, dicalcium phosphate, tribasic calcium phosphate, calcium sulfate, dextrin/dextrates, maltodextrin, compressible sugars, confectioner's sugar, glyceryl palmitostearate, hydrogenated vegetable oil, kaolin, maltodextrin, polymethacrylates, potassium chloride, sodium chloride, sucrose, sugar spheres and talc.

In accordance with the invention, when the ingredients are incorporated prior to granulation, they are referred to as "intragranular", i.e., within the granule. When the ingredients are incorporated after granulation, they are referred to as "extragranular".

One aspect of the invention provides a granule comprising atazanavir sulfate and an intragranular lubricant, said granule having an interior section and an exterior surface and wherein at least a portion of the intragranular lubricant is present in the interior section of the granule, i.e., within the granule. The interior section of the granule is defined by a space having a volume within the granule. Typically, the volume of the space is at least 10% of the total volume of the granule, more typically at least 50% of the total volume of the granule, and even more typically at least 80% of the total volume of the granule. For purposes of clarification, the space occupies by the internal section of the granule is not to be confused with empty space. It is occupies by the atazanavir sulphate, intragranular lubricant, and optionally other ingredients.

Typically, the granule comprises from about 0.1 to 15% of the intragranular lubricant, more typically from about 1 to 5% of the intragranular lubricant based on the total weight of the granule.

Typically the granule comprises from about 10 to 99.9% of the atazanavir sulphate, more typically from about 30 to 90% of the atazanavir sulfate based on the total weight of the granule.

The granule may further comprise, for example, from about 1 to 20%, based on the total weight of the granule, of a disintegrant.

The granule may optionally further comprise, for example, from about 0 to 20%, based on the total weight of the granule, of a binder.

The granule may further comprising, for example, from about 1 to 20%, based on the total weight of the granule, of a filler.

The present invention further encompasses a composition comprising a plurality of the granules. Such a composition may exist in containers, for example, when the granules are prepared in one manufacturing location and tableted in another location.

In one aspect of the invention, there is provided a compressed tablet comprising granules containing atazanavir sulfate and an intragranular lubricant, said granules having an interior section and an exterior surface and wherein at least a portion of the intragranular lubricant is present in the interior section of the granules.

Typically, the compressed tablet comprises from about 0.1 to 10% of the intragranular lubricant, more typically from about 0.5 to 8% of the intragranular lubricant, based on the total weight of the compressed tablet.

Typically, the compressed tablet comprises about 10 to 99.9% of the atazanavir sulphate, more typically from about 30 to 90% of the atazanavir sulphate, based on the total weight of the compressed tablet. The compressed tablet comprises a therapeutically effective amount of atazanavir, present as atazanavir sulphate. The term "therapeutically effective amount" means the total amount of each active component that is sufficient to show a meaningful patient benefit, e.g., a sustained reduction in viral load. In general, the goals of treatment are suppression of viral load, restoration and preservation of immunologic function, improved quality of life, and reduction of HIV-related morbidity and mortality. When applied to an individual active ingredient, administered alone, the term refers to that ingredient alone. When applied to a combination, the term refers to combined amounts of the active ingredients that result in the therapeutic effect, whether administered in combination, serially or simultaneously. The term "patient" includes both human and other mammals. The typical dose of atazanavir to be administered to patients, for example human beings of approximately 70 kilograms ("kg") body weight, is from about 3 miligrams ("mg") to about 1.5 grams ("g"), preferably from about 10 mg to about 1.25 g, for example from about 50 mg to about 600 mg per person per day, divided preferably into 1 to 4 single doses which may, for example, be of the same size. Usually, children receive half of the adult dose. The present invention also encompasses treating an HIV infection in a patient, comprising administering to the patient a therapeutically effective amount of a compressed tablet of the invention.

Typically, the compressed tablet comprises from about 1 to 20%, more typically from about 2 to 12%, based on the total weight of the compressed tablet, of a disintegrant.

Typically, the compressed tablet comprises from about 0 to 10%, more typically from about 0.2 to 6%, based on the total weight of the compressed tablet, of a binder.

Typically, the compressed tablet comprises from about 5 to 90%, more typically from about 15 to 40%, based on the total weight of the compressed tablet, of a filler.

Typically, the compressed tablet comprises from about 0.1 to 3%, more typically from about 0.2 to 1.5%, based on the total weight of the compressed tablet, of an extragranular lubricant.

Examples of the intragranular lubricant in this aspect are selected from stearic acid, silicon dioxide and mixtures thereof. An example of the extragranular lubricant is magnesium stearate.

The compressed tablets of the present invention can also be film coated. Film coat concentration can be varied up to about 10% to complement the drug amount, and preferably about 2.5 to about 3.5%. Typical film coating suspensions include combinations of one, two or three of the following components: carboxymethylcellulose sodium, carnauba wax, cellulose acetate phthalate, cetyl alcohol, confectioner's sugar, ethyl cellulose, gelatin, hydroxyethyl cellulose, hydroxypropyl cellulose, hydroxypropyl methylcellulose, liquid glucose, maltodextrin, methyl cellulose, microcrystalline wax, Opadry and Opadry II, polymethacrylates, polyvinyl alcohol, shellac, sucrose, talc, titanium dioxide, and zein.

In another aspect of the invention, one or more other agents having anti-HIV activity is included in the compressed tablet. As used herein, the term "anti-HIV activity" means the agent has efficacy against the HIV virus. Other agents may be selected, for example, from the group consisting of nucleoside HIV reverse transcriptase inhibitors, non-nucleoside HIV reverse transcriptase inhibitors, HIV protease inhibitors, HIV fusion inhibitors, HIV attachment inhibitors, CCR5 inhibitors, CXCR4 inhibitors, HIV budding or maturation inhibitors, and HIV integrase inhibitors.

Another aspect of the invention is the compressed tablet wherein the other agent is a nucleoside HIV reverse transcriptase inhibitor selected from the group consisting of didanosine, emtricitabine, lamivudine, stavudine, tenofovir, zalcitabine, and zidovudine, or a pharmaceutically acceptable salt thereof. A perferred combination with atazanavir is wherein the other agents are tenofovir disoproxil fumarate and emtricitabine. A typical dosage for the drug Truvada™ (emtricitabine - tenofovir disoproxil fumarate) is emtricitabine 200 mg plus tenofovir 300 mg one tablet once per day. A typical dosage for the drug Epzicom™ (abacavir-lamivudine) is abacavir sulfate 600 mg and lamivudine 300 mg. Suitable dosages for combination therapy with atazanavir can be determined by those skilled in the art.

Another aspect of the invention is the compressed tablet wherein the other agent is a non-nucleoside HIV reverse transcriptase inhibitor selected from the group consisting of delavirdine, efavirenz, nevirapine and UK 4530C 1 or a pharmaceutically acceptable salt thereof.

Another aspect of the invention is the compressed tablet wherein the other agent is a HIV protease inhibitor selected from the group consisting of amprenavir, indinavir, lopinavir, nelfinavir, ritonavir, saquinavir and fosamprenavir, or a pharmaceutically acceptable salt thereof. Ritonavir is a preferred drug to be used in combination with atazanavir sulfate as another agent having anti-HIV activity. However, ritonavir is more commonly used as a boosting agent for another drug, e.g., atazanavir. When given as a protease inhibitor booster, the dosing typically ranges from 100-400 mg twice daily or, if used as a part of a once-daily regimen, 100-200 mg once-daily.

Another aspect of the invention is the compressed tablet wherein the other agent is a HIV fusion inhibitor selected from enfuvirtide or T-1249, or a pharmaceutically acceptable salt thereof.

Another aspect of the invention is the compressed tablet wherein the other agent is a CCR5 inhibitor selected from the group consisting of maraviroc, Sch-C, Sch-D, TAK-220, PRO-140, PF-232798 and UK-427,857, or a pharmaceutically acceptable salt thereof.

Another aspect of the invention is the compressed tablet wherein the other agent is the CXCR4 inhibitor AMD-3100 or a pharmaceutically acceptable salt thereof.

Another aspect of the invention is the compressed tablet wherein the other agent is the budding or maturation inhibitor PA-457, or a pharmaceutically acceptable salt thereof.

Another agent is the integrase inhibitor raltegravir, or a pharmaceutically acceptable salt thereof. The chemical name of the potassium salt is N-[(4-fluorophenyl)methyl]-1,6-dihydro-5-hydroxy-1-methyl-2-[1-methyl-1-[[(5-methyl-1,3,4-oxadiazol-2-yl)carbonyl]amino]ethyl]-6-oxo-4-pyrimidinecarboxamide monopotassium salt. Raltegravir is described, for example, in WO 2003/035077 published May 1, 2003 and Drugs of the Future 2007, 32(2): 118-122, Y Wang., et al. Typical dosages for raltegravir in monotherapy are 100, 200, 400, and 600 mg given twice daily. Suitable dosages for combination therapy with atazanavir can be determined by those skilled in the art.

Table 1 includes some agents useful in treating AIDS and HIV infection which may by suitable for use in accordance with this invention as the other agents having anti-HIV activity, as well as other drugs that may be co-administered.

**Table 1. ANTIVIRALS**

| Drug Name | Manufacturer | Indication |
|---|---|---|
| 097 (non-nucleoside reverse transcriptase inhibitor) | Hoechst/Bayer | HIV infection, AIDS, ARC |
| Amprenavir 141 W94 GW 141 (protease inhibitor) | Glaxo Wellcome | HIV infection, AIDS, ARC |
| Abacavir (1592U89) GW 1592 (RT inhibitor) | Glaxo Wellcome | HIV infection, AIDS, ARC |
| Acemannan | Carrington Labs (Irving, TX) | ARC |
| Acyclovir | Burroughs Wellcome | HIV infection, AIDS, ARC, in combination with AZT |
| AD-439 | Tanox Biosystems | HIV infection, AIDS, ARC |
| AD-519 | Tanox Biosystems | HIV infection, AIDS, ARC |
| Adefovir dipivoxil AL-721 | Gilead Sciences Ethigen CA) (Los Angeles, CA) | HIV infection, ARC, PGL HIV positive, AIDS PGL HIV positive, AIDS |
| Alpha Interferon HIV in combination w/Retrovir | Glaxo Wellcome | Kaposi's sarcoma |
| Ansamycin LM 427 | Adria Laboratories (Dublin, OH) Erbamont (Stamford, CT) | ARC |
| Antibody which Neutralizes pH Labile alpha aberrant Interferon | Advanced Biotherapy Concepts (Rockville, MD) | AIDS, ARC |
| Ar177 | Aronex Pharm | HIV infection, AIDS, ARC |
| Beta-fluoro-ddA | Nat'1 Cancer Institute | AIDS-associated diseases |
| BMS-232623 (CGP-73547) (protease inhibitor) | Bristol-Myers Squibb/ qui Novartis | AIDS HIV infection, AIDS, ARC |
| (CGP-61755) (protease inhibitor) | Bristol-Myers Squibb/ Novartis | HIV infection, AIDS, ARC |
| CI-1012 | Warner-Lambert | HIV-1 infection |
| Cidofovir | Gilead Science | CMV retinitis, herpes, papillomavirus |
| Curdlan sulfate | AJI Pharma USA | HIV infection |
| Cytomegalovirus Immune globin | MedImmune | CMV retinitis CMV retinitis |
| Cytovene | Syntex | Sight threatening |
| Ganciclovir | | CMV peripheral, CMV retinitis |
| Delaviridine (RT inhibitor) | pharmacia-Upjohn | HIV infection, AIDS, ARC |
| Dextran Sulfate | Ueno Fine Chem. Ind. Ltd. (Osaka, Japan) | AIDS, ARC, HIV positive asymptomatic |
| ddC Dideoxycytidne | Hoffman-La Roche | HIV infection, AIDS, ARC |
| ddI Dideoxyinosine | Bristol-Myers Squibb | HIV infection, AIDS, ARC; combination with AZT/d4T |
| DMP-450 (protease inhibitor) | AVID (Camden, NJ) | HIV infection, AIDS, ARC |
| Efavirenz (DMP 266) (-)6-Chloro-4-(S)-cyclopropylethynyl-4(S)-trifluoro-methyl-1,4-dibydro-2H-3,1-benzoxazin-2-one, STOCRINE (non-nucleoside RT inhibitor) | DuPont Merck | HIV infection, AIDS, ARC |
| EL10 | Elan Corp, PLC GA) (Gainesville, GA) | HIV infection HIV infection |
| Emtricitabine (Emtriva^{®}) (reverse transcriptase inhibitor) | Gilead | HIV infection, AIDS |
| Famciclovir | Smith Kline | herpes zoster, herpes simplex |
| FTC (reverse transcriptase inhibitor) | Emory University | HIV infection,AIDS, ARC |
| GS 840 (reverse transcriptase inhibitor) | Gilead | HIV infection, AIDS, ARC |
| HBY097 (non-nucleoside reverse transcriptaseinhibitor) | Hoechst Marion Roussel | HIV infection, AIDS, ARC |
| Hypericin | VIMRx Pharm. | HIV infection, AIDS, ARC |
| Recombinant Human Interferon Beta | Triton Biosciences (Almeda, CA) | AIDS, Kaposi's sarcoma, ARC |
| Interferon alfa-n3 | Interferon Sciences | ARC, AIDS |
| Indinavir | Merck | HIV infection, AIDS, ARC, asymptomatic HIV positive, also in combination with AZT/ddI/ddC |
| ISIS 2922 | ISIS Pharmaceuticals | CMV retinitis |
| KNI-272 | Nat'1 Cancer Institute | HIV-associated diseases |
| Lamivudine, 3TC (reverse transcriptase inhibitor) | Glaxo Wellcome | HIV infection, AIDS ARC, also with AZT |
| Lobucavir | Bristol-Myers Squibb | CMV infection |
| Nelfinavir (protease inhibitor) | Agouron Pharmaceuticals | HIV infection, AIDS, ARC |
| Nevirapine (RT inhibitor) | Boeheringer Ingleheim | HIV infection, AIDS, ARC |
| Novapren | Novaferon Labs, Inc. (Akron, OH) | HIV inhibitor |
| Peptide T Octapeptide Sequence | Labs Peninsula (Belmont, CA) | AIDS |
| Trisodium Phosphonoformate | Astra Pharm. Products, Inc. Products, | CMV retinitis, HIV infection, other CMV infections |
| PNU-140690 (protease inhibitor) | pharmacia Upjohn | HIV infection, AIDS, ARC |
| Probucol | Vyrex | HIV infection, AIDS |
| RBC-CD4 | Sheffield Med. Tech (Houston, TX) | HIV infection, AIDS, ARC |
| Ritonavir (protease inhibitor) | Abbott | HIV infection, AIDS, ARC |
| Saquinavir (protease inhibitor) | Hoffmann-LaRoche | HIV infection, AIDS, ARC |
| Stavudine; d4T Didehydrodeoxy-thymidine | Bristol-Myers Squibb | HIV infection, AIDS, ARC |
| Valaciclovir | Glaxo Wellcome | Genital HSV & CMV infections |
| Virazole Ribavirin | Viratek/ICN (Costa Mesa, CA) | asymptomatic HIV-positive, LAS, ARC |
| VX-478 | Vertex | HIV infection, AIDS, ARC |
| Zalcitabine | Hoffmann-LaRoche | HIV infection, AIDS, ARC, with AZT |
| Zidovudine; AZT | Glaxo Wellcome | HIV infection, AIDS, ARC, Kaposi's sarcoma, in combination with other therapies |
| Tenofovir disoproxil, fumarate salt (Viread^{®}) (reverse transcriptase inhibitor) | Gilead | HIV infection, AIDS |
| Combivir^{®} (reverse transcriptase inhibitor) | GSK | HIV infection, AIDS |
| abacavir succinate (or Ziagen^{®}) (reverse transcriptase inhibitor) | GSK | HIV infection, AIDS |
| Fuzeon (Enfuvirtide, T-20) | Roche/Trimeris | HIV infection, AIDS, viral fusion inhibitor |
| Trizivir^{®} | | HIV infection, AIDS |
| Kaletra^{®} | Abbott | HIV infection, AIDS, ARC |

| IMMUNOMODULATORS | | |
|---|---|---|
| Drug Name | Manufacturer | Indication |
| AS-101 | *Wveth-Averst* | AIDS |
| Bropirimine | Pharmacia Upjohn | Advanced AIDS |
| Acemannan | Carrington Labs, Inc. (Irving, TX) | AIDS, ARC |
| CL246,738 | American Cyanamid Lederle Labs | AIDS, Kaposi's sarcoma |
| EL 10 | Elan Corp, PLC (Gainesville, GA) | HIV infection |
| FP-21399 | Fuki ImmunoPharm | Blocks HIV fusion with CD4+ cells |
| Gamma Interferon | Genentech | ARC, in combination w/TNF (tumor necrosis factor) |
| Granulocyte Macrophage Colony Stimulating Factor | Genetics Institute Sandoz | AIDS |
| Granulocyte Macrophage Colony Stimulating, Factor | Hoechst-Roussel Immunex | AIDS |
| Granulocyte Macrophage Colony Stimulating Factor | Schering-Plough | AIDS combination w/AZT |
| HIV Core Particle Immunostimulant | Rorer | Seropositive HIV |
| IL-2 Interleukin-2 | Cetus | AIDS, in combination w/AZT |
| IL-2 Interteukin-2 | Hoffman-LaRoche Immunex | AIDS, ARC, HIV, in combination w/AZT |
| IL-2 Interteukin-2 (aldeslukin) | Chiron | AIDS, increase in CD4 cell counts |
| Immune Globulin Intravenous (human) | Cutter Biological (Berkeley, CA) | Pediatric AIDS, in combination w/AZT |
| IMREG-1 | Imreg (New Orleans, LA) | AIDS, Kaposi's sarcoma, ARC, PGL |
| IMREG-2 | Imreg (New Orleans, LA) | AIDS, Kaposi's sarcoma, ARC, PGL |
| Imuthiol Diethyl Dithio Carbamate | Merieux Institute | AIDS, ARC |
| Alpha-2 Interferon | Schering Plough | Kaposi's sarcoma w/AZT, AIDS |
| Methionine-Enkephalin | TNI Pharmaceutical (Chicago, IL) | AIDS, ARC |
| MTP-PE Muramyl-Tripeptide Granulocyte Colony Stimulating Factor | Ciba-Geigy Corp. Amgen | Kaposi's sarcoma AIDS, in combination w/AZT |
| Remune | Immune Response | Immunotherapeutic |
| rCD4 Recombinant Soluble Human CD4 | Genentech | AIDS, ARC |
| rCD4-IgG hybrids | | AIDS, ARC |
| Recombinant Soluble Human CD4 | Biogen | ARC AIDS, ARC |
| Interferon Alfa 2a | Hoffman-La Roche in combination w/AZT | Kaposi's sarcoma, AIDS, ARC |
| SK&F106528 Soluble T4 | Smith Kline | HIV infection |
| Thymopentin | Immunobiology Research Institute (Annandale, NJ) | HIV infection |
| Tumor Necrosis Factor; TNF | Genentech | ARC, in combination w/gamma Interferon |

| ANTI-INFECTIVES | | |
|---|---|---|
| Drug Name | Manufacturer | Indication |
| Clindamycin with Primaquine | Pharmacia Upjohn | PCP |
| Fluconazole | Pfizer | Cryptococcal meningitis, candidiasis |
| Pastille Nystatin Pastille | Squibb Corp. | Prevention of oral candidiasis |
| Ornidyl Eflornithine | Dow | PCP |
| Pentamidine Isethionate (IM & IV) | LyphoMed (Rosemont, IL) | PCP treatment |
| Trimethoprim | | Antibacterial |
| Trimethoprim/sulfa | | Antibacterial |
| Piritrexim | Burroughs Wellcome | PCP treatment |
| Pentamidine Isethionate for Inhalation | Fisons Corporation | PCP prophylaxis |
| Spiramycin | Rhone-Poulenc diarrhea | Cryptosporidial |
| Intraconazole-R51211 | Janssen-Pharm. | Histoplasmosis; cryptococcal meningitis |
| Trimetrexate | Warner-Lambert | PCP |
| Daunorubicin | NeXstar, Sequus | Kaposi's sarcoma |
| Recombinant Human Erythropoietin | Ortho Pharm. Corp. | Severe anemia assoc. with AZT therapy |
| Recombinant Human Growth Hormone | Serono | AIDS-related wasting, cachexia |
| Megestrol Acetate | Bristol-Myers Squibb | Treatment of anorexia assoc. W/AIDS |
| Testosterone | Alza, Smith Kline | AIDS-related wasting |
| Total Enteral Nutrition | Norwich Eaton Pharmaceuticals | Diarrhea and malabsorption related to AIDS |

Abacavir sulfate may be included within the same phase as the atazanavir sulfate or its formulation, i.e., as a monolithic tablet, or it may be included within another phase, i.e., a multi-layer tablet. When included in a monolithic tablet, abacavir sulfate may be blended intragranularly with the atazanavir sulfate or its formulation or added extragranularly. When included in a multi-layer tablet, the atazanavir sulfate is in one layer and the abacavir sulfate is in another layer, e.g., bilayer. Alternatively, when more than one other agent having anti-HIV activity is combined with atazanavir sulfate, e.g, ritonavir, emtricitabine and tenofovir, in a multilayer tablet, it may be desireable to separate certain agents by incorporating them in separate layers.

The granules may be prepared by a process for preparing granules comprising:
(a) blending atazanavir sulfate and an intragranular lubricant to for a first blend;
(b) granulating (e.g., by wet granulation) the first blend in the presence of a fluid (e.g., water, ethanol, solution of hydroxypropyl cellulose, foam of hydroxypropyl cellulose, solution of povidone) to form wet granules;
(c) removing at least a portion of the liquid from the wet granules to form dry granules. Typically, the process further comprises sizing (e.g., milling) the dry granulate to form sized granules, compressing the sized granules into a compressed tablet and coating the compressed tablet with a film coating to form a coated, compressed tablet.

Wet granulation can be conducted, for example, using granulator mixers, such as a Fielder 10 L high shear granulator mixer, a low shear, a drum or pan granulator, and a fluid bed granulator. Granulation can also be achieved by conducting dry granulation (without fluid) using a roller compaction process. One preferred technique to conduct the granulation step in accordance with the present invention is to utilize an aqueous air foam such as described in U.S. Pat. No. 7,011,702 issued March 14, 2006. The drying step can be conducted, for example, using a Glatt WSG-15 fluid bed drier or a tray drier. The sizing (e.g., milling) step can be conducted, for example, using mills such as a Comil or a Fitz mill. The blending steps can be conducted in a V-blender or a ribbon blender. The compression step to form the tablet can be done, for example, using a variety of presses including a beta press, single station F-press or a 6-station Korsh. Film coating can be performed, for example, in a Glatt Column coater or a smaller Hi-coater (9" 12" pan).

The following examples are referential examples.

### Reference EXAMPLE 1

### 1-[4-(Pyridin-2-yl)phenyl]-5(S)-2,5-bis{[N-(methoxycarbonyl)-L-tert-leucinyl]amino}-4-(S)-hydroxy-6-phenyl-2-azahexane, Sulfate salt (Form A) (Atazanavir sulfate-Form A)

### A.

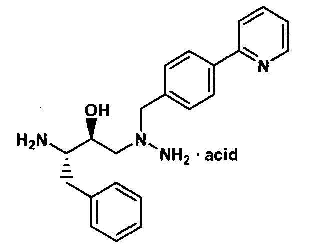

### (1-[4-(Pyridin-2-yl)phenyl]-5(S)-2,5-bis[tert-butyloxycarbonyl)amino]-4(S)-hydroxy-6-phenyl-2-azahexane.3HCl (Triamine.3HCl Salt))

To a 1000 mL, 3-neck, round-bottom flask fitted with mechanical stirrer, nitrogen inlet and temperature probe is added the protected triamine 1-[4-(pyridin-2-yl)phenyl]-5(S)-2,5-bis[tert-butyloxycarbonyl)amino]-4(S)-hydroxy-6-phenyl-2-azahexane 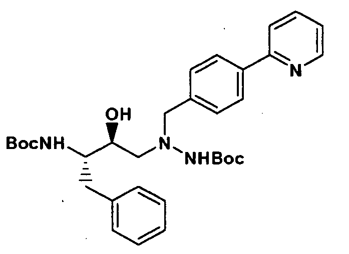 (100 g, 0.178 mol), and CH₂Cl₂ (500 mL; 5 mL/g of protected triamine input) (prepared as described in Z. Xu et al., Process Research and Development for an Efficient Synthesis of the HIV Protease Inhibitor BMS-232,632, Organic Process Research and Development, 6, 323-328 (2002)) and the resulting slurry is agitated while maintaining the temperature at from about 5 to about 22°C.

Concentrated hydrochloric acid (68 mL, 0.82 mole, 4.6 eq.) is added to the reaction mixture at a rate such that the temperature of the reaction mixture remained between 5 and 30°C. The reaction mixture is heated to 30 to 40°C and agitated until the reaction is judged complete by HPLC assay.

Water is added (70-210 mL, 0.7-2.1 mL/g protected triamine input) to the reaction mixture, the reaction mixture is agitated for 15 minutes and the phases were allowed to separate. The upper, product (triamine.3HCl salt)-rich aqueous oil is transferred to an addition funnel.

### B.

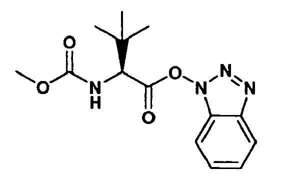

**(Active Ester of N-methoxycarbonyl-L-tert-leucine**

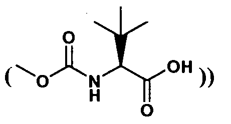

To a 3000 mL, 3-neck round bottom flask fitted with mechanical stirrer, addition funnel, nitrogen inlet, and temperature probe is added N-methoxycarbonyl-L-tert-leucine (77.2g, 0.408 mol, 2.30 eq.), 1-hydroxybenzotriazole (HOBT) (60.8 g, 0.450 mol, 2.53 eq.), and N-ethyl N'-dimethylaminopropyl carbodiimide (EDAC) (82.0 g, 0.430 mol, 2.42 eq.), followed by CH₂Cl₂ (880 mL; 8.8 mL/g of protected triamine input) and the mixture is stirred at ambient temperature (18-25°C) until formation of the active ester is complete, as judged by HPLC.

### C. 1-[4-(Pyridin-2-yl)phenyl]-5(S)-2,5-bis{[N-(methoxycarbonyl)-L-tert-leucinyl]amino}-4(S)-hydroxy-6-phenyl-2-azahexane (atazanavir free base)

Anhydrous dibasic potassium phosphate (K₂HPO₄; 226 g., 1.30 mol, 7.30 eq. wrt protected triamine) is dissolved in 1130 mL of water (11.3 mL/g of protected amine; 5 mL/g of K₂HPO₄).

The K₂HPO₄ solution is added to the active ester solution prepared in Part B. To the stirred active ester/aqueous K₂HPO₄ mixture is slowly added the aqueous solution of Part A hydrogen chloride salt over a period of 1.5 to 2.0 h while maintaining agitation and a pot temperature between 5 and 20°C.

After the addition of the solution of the Part A hydrogen chloride salt is complete, the reaction mixture (coupling reaction) is heated to 30-40°C and agitated until the coupling reaction is judged complete by HPLC assay.

The coupling mixture is cooled to 15 to 20°C and the lower, product rich organic phase is separated from the upper, spent aqueous phase.

The product rich organic phase is washed with 1M NaH₂PO₄ (880 mL; pH=1.5; 8.8 mL/g of protected triamine input; 5 mole eq. wrt protected triamine), the phases were allowed to separate, and the spent aqueous phase is removed.

The washed product rich organic phase is stirred with 0.5 N NaOH (800 mL; 8 mL/g of protected triamine input) until HPLC assay of the rich organic phase showed the active esters to be below 0.3 I.I. each. The phases were allowed to separate and the spent aqueous phase is removed.

The rich organic phase is washed with 5% NaH₂PO₄ (450 mL, 4.5 mL/g of protested triamine input; pH=4.3), the phases were allowed to separate and the spent aqueous phase is removed.

The rich organic phase is washed with 10 w/v% NaCl (475 mL, 4.75 mL/g of protected triamine input) and the spent aqueous phase is removed.

The concentration of title free base in solution is 120 to 150 mg/mL with an in-process calculated yield of 95-100 mol%.

### D. Solvent Exchange from CH₂Cl₂ into Acetone/N-Methylpyrrolidone

To the rich Part C free base solution in a 3000 mL, 3-neck round-bottom flask fitted with mechanical stirrer, temperature probe, and distillation condenser, is added N-methylpyrrolidone (148 mL; 1.25 mL/g of Part C free base based on in-process quantification assay). The solution is concentrated to ca. 360 mL (2.5-3.5 mL/g of Part C free base) using a jacket temperature of 70°C or less; 500 mL of acetone (4-5 mL/g of Part C free base) is added to the concentrated solution and the mixture is distilled to a volume of about 400 mL or less.

The acetone addition and distillation were repeated until in-process assay indicated the CH₂Cl₂ level had reached the target endpoint. At crystallization volume, the CH₂Cl₂ content in the rich organic solution is 0.77 v/v %. Acetone is added to the concentrated free base solution to reach a total solution of 16 mL/g of free base. The bath temperature is maintained at 40-50°C to prevent crystallization of free base. The solution is polish filtered through a 10-micron or finer filter while maintaining the temperature at 40 to 50°C. The polish filter is rinsed with acetone (125 mL, 1.0 mL/g of free base) and the rinse is added to the rich free base acetone/N-methylpyrrolidone solution which is used in the next step.

### E. 1-[4-(Pyridin-2-yl)phenyl]-5(S)-2,5-bis{[N-(methoxycarbonyl)-L-tert-leucinyl]amino}-4(S)-hydroxy-6-phenyl-2-azahexane sulfate salt

About 10% (2 g) of the total charge of concentrated sulfuric acid (19 g, 1.10 eq.) is added to the free base acetone/N-methylpyrrolidone solution of Part D, while maintaining the temperature at 40-50°C, via subsurface addition.

The reaction mixture is seeded with 5.0 wt % (wrt calculated free base in solution) of sulfate salt. The seeded mixture is agitated at 40-50°C for at least 30 minutes during which time the sulfate salt began crystallizing as evidenced by the mixture increasing in opacity during this time.

The remaining sulfuric acid (17.8 g) is added over *ca.* 5 h in five stages according to the following protocol, defined by a cubic equation, while keeping the temperature at 40-50°C.
The rate of each addition stage is determined according to the cubic equation described in U.S. Patent Publication No. US20050256202A1, published November 17,2005

After addition of H2_{S}O4 is complete, the slurry is cooled to 20-25°C for at least I h with agitation. The slurry is agitated at 20-25°C for at least 1 h. The sulfate salt is filtered and the mother liquor is recycled as needed to effect complete transfer. The filter cake is washed with acetone (5-10 mL/g of free base; 1200 mL acetone). The sulfate salt is dried at NMT 55°C under vacuum until the LOD <1% to produce a crystalline material.

Further details on the preparation and chatacterization of this compound are disclosed in U.S. Patent Publication No. US20050256202A1, published November 17,2005.

### Reference EXAMPLE 2

### Atazanavir Sulfate - Pattern C Material

### Method A:

Form A crystals of atazanavir sulfate (prepared as described in Example 1) (25.33 g) were suspended in 200 mL of water and the mixture is stirred mechanically to produce a thick gel which is dried.

The dried mixture is ground with a spatula to produce Pattern C material.

Further details on the preparation and chatacterization of this compound are disclosed in U.S. Patent Publication No. US20050256202A1, published November 17,2005.

### Method B:

Form A crystals of atazanavir sulfate is wet granulated using a sufficient amount of water (about 40% w/w) in a suitable mixer-granulator. The wet mass is dried in an oven. The product is sized using a suitable screen.

Further details on the preparation and chatacterization of this compound are disclosed in U.S. Patent Publication No. US20050256202A 1, published November 17, 2005.

### Reference EXAMPLE 3

### Atazanavir Sulfate - Form E3 (Triethanol Solvate)

Atazanavir free base (prepared as described in Example 1, Part C) (3.0 g, 4.26 mmol) is slurried in dry, 200 proof ethanol (20.25 mL, 6.75 mL/g of free base) in a 100 mL, 3-neck round-bottom flask fitted with a mechanical stirrer, temperature probe, and a pressure-equalizing liquid addition funnel.

Concentrated H₂SO₄ (0.25 mL, 0.46 g, 4.69 mmol, 1.1 eq.) is added to the slurry of atazanavir free base which is maintained at 20-25°C. The resulting solution (KF of 0.2 to 1.0% water) is polish filtered (Whatman #1 paper), the filter rinsed with 2.25 mL of absolute ethanol and the rinse added to the filtered solution. The solution is heated to 37°C and seeded with 10 mg of amorphous atazanavir sulfate derived from Form E3 crystals (by exposing Form E3 crystals to ambient temperature), and the mixture is agitated for 15 min. Heptane (380 mL, 8.25 mL/g of free base) is added over 1 hour. The resulting crystallization mixture is agitated for 8 h at 15-25°C. Crystallized atazanavir sulfate is filtered on a Büchner funnel. The product cake is washed with 184 mL (4 mL/g of free base) of 1:1 ethanol : heptane. The product cake is washed with 46 mL (1 mL/g of free base) of heptane. The resulting product is dried under vacuum at 40-50°C until it had an LOD = 0.97%.

Further details on the preparation and chatacterization of this compound are disclosed in U.S. Patent Publication No. US20050256202A1, published November 17, 2005.

### Reference EXAMPLE 4

### Atazanavir Sulfate Tablets

For use in the remaining examples, atazanavir sulfate was prepared following procedures substantially as described in Examples 1-3.

A compressed tablet having a dose of 300 mg (as Free Base) was prepared having the following composition.

| Ingredient | | % (w/w) of the final composition |
|---|---|---|
| Atazanavir (as sulfate salt) | Intragranular | 56.9 |
| Stearic acid | | 2.8 |
| Microcrystalline cellulose | | 7.4 |
| Sodium starch glycolate | | 1.4 |
| Crospovidone | | 1.4 |
| HPC | | 0.7 |
| Microcrystalline cellulose | Extragranular | 23.65 |
| Sodium starch glycolate | | 3 |
| Crospovidone | | 2 |
| Magnesium stearate | | 0.75 |

| | | |
|---|---|---|
| Coating: Opadry II, based on 3% coat | | |

The preparation of the atazanavir sulfate tablets was commenced by blending the intragranular ingredients in a tumbling type blender, e.g., V-blender, in a 3-step process. Firstly, a portion of atazanavir sulfate (12% of total atazanavir sulfate weight) and stearic acid was blended for 125 revolutions. Secondly, the remaining atazanavir sulfate was added and blended for another 250 revolutions. Thirdly, microcrystalline cellulose, sodium starch glycolate and crospovidone were added and the mixture was further blended for 250 revolutions.

The intragranular blend was transferred to a high shear mixer, e.g., 65L Diosna or Glatt-Fuji. A hydroxypropyl cellulose ("HPC") solution was prepared and transferred to a foam generator (Dow Chemical Company) to make HPC foam. Foam quality (expressed as: (volume of air-volume of HPC solution)/(volume of air)X 100) was greater than 70%. HPC weight ranged from 0.5 - 3 %w/w of dry weight of intragranular blend, water to make the HPC solution ranged from 30 - 38%w/w of dry weight of intragranular blend. Granulation of the intragranular powder with HPC foam was conducted at the following mixing speeds: 90 - 200 RPM impeller speed (depending on batch size and high-shear mixer type), 1300-1770 RPM chopper speed. After completion of addition of calculated amount of HPC solution as foam, wet-massing was conducted without stopping the high-shear mixer. Wet massing time ranged from 0.5 - 2 min.

The wet granulation was transferred to a fluid-bed dryer and dried to a level where loss on drying was not more than 4.5%w/w.

The dried granulation was sized through a 1 millimeter ("mm") screen.

The milled granulation was blended with calculated extragranular microcrystalline cellulose, sodium starch glycolate and crospovidone in a tumbling type blender for 250 revolutions. Magnesium stearate was then added to the blend and blended for 75 revolutions.

The resulting final blend was then tableted to obtain a desired tablet weight and hardness (typical according to USP General Chapters: <1216> Tablet Friability). The final blend can also be used to manufacture any other oral dosage form such as capsules, granule powders or pellets.

An Opadry II coating suspension (18%w/w solids) was prepared to coat the tablets. The suspension was continuously stirred during the coating process. A coater (Glatt, Thomas Engineering, or Vector) was used to coat the tablets to a tablet weight gain of 2 - 3.5 %w/w, which was sufficient.

The so-formed atazanavir sulfate film coated tablets had an excellent release profile, about 95% after 45 minutes, which is similar to Reyataz (atazanavir sulfate) capsules, according to USP General Chapters: <1092> THE DISSOLUTION PROCEDURE: DEVELOPMENT AND VALIDATION, in vitro dissolution profiles of immediate-release products typically show a gradual increase reaching 85% to 100% at about 30 to 45 minutes.

Alternative procedures for making the tablets include, for example:
A) Procedure of blending intragranular ingredients:
   1. A 2-step mixing process in a tumbling type blender. Firstly, a portion of atazanavir sulfate (50% of total atazanavir sulfate weight) and stearic acid was blended for 5 - 15 min. Secondly, all the remaining atazanavir sulfate, microcrystalline cellulose, sodium starch glycolate and crospovidone were added to the atazanavir sulfate/stearic acid mixture and blended for 10 min.
   2. A high shear mixing process. Firstly, a portion of atazanavir sulfate was blended with stearic acid in a suitable size high-shear mixer (50 - 350 RPM impeller speed). Then, all the remaining atazanavir sulfate, microcrystalline cellulose, sodium starch glycolate and crospovidone were added to the atazanavir sulfate/stearic acid mixture and blended. Alternatively, all the ingredients were added and blended in the high-shear mixer in 1 step.
B) Incorporation of HPC
   1. HPC was added as dry powder and mixed with other ingredients in the intragranular blend. Water, instead of HPC foam, was added during granulation.
   2. HPC was dissolved in water, and HPC solution was added during granulation.
C) The granulation was also dried using a tray-oven.

### Reference EXAMPLE 5

### Atazanavir Sulfate Tablets

A compressed tablet having a dose of 300 mg (as Free Base) was prepared having the following composition.

| Ingredient | | % (w/w) of the final composition |
|---|---|---|
| Atazanavir (as salt) | Intragranular | 57.0 |
| Stearic Acid | | 2.8 |
| Microcrystalline cellulose | | 7.3 |
| Sodium starch glycolate | | 1.4 |
| Crospovidone | | 2.1 |
| Povidone | | 0.2 |
| Microcrystalline cellulose | Extragranular | 25.2 |
| Crospovidone | | 3.0 |
| Magnesium Stearate | | 1.0 |

| | | |
|---|---|---|
| Coating: Opadry II, based on 3% coat | | |

The preparation of atazanavir tablets was commenced by blending the intragranular ingredients in a tumbling type blender, e.g., V-blender, in a 3-step process. Firstly, a portion of atazanavir sulfate (12% of total atazanavir sulfate weight) and stearic acid was blended for 125 revolutions. Secondly, the remaining atazanavir sulfate was added and blended for another 250 revolutions. Thirdly, microcrystalline cellulose, sodium starch glycolate, crospovidone and povidone were added and the mixture was further blended for 250 revolutions.

The intragranular blend was transferred to a high shear mixer, e.g., 65L Diosna or Glatt-Fuji. Granulation of the intragranular powder with water was conducted at the following mixing speeds: 90 - 200 RPM impeller speed (depending on batch size and high-shear mixer type), 1300 - 1770 RPM chopper speed. After completion of addition of calculated amount of water, wet-massing was conducted without stopping the high-shear mixer. Wet massing time ranged from 0.5 - 2 min.

The wet granulation was transferred to a fluid-bed dryer and dried to a level where loss on drying was not more than 4.5%w/w.

The dried granulation was sized through a 1 mm sized screen.

The milled granulation was blended with calculated extragranular microcrystalline cellulose and crospovidone in a tumbling type blender for 250 revolutions. Magnesium stearate was then added to the blend and blended for 75 revolutions.

The resulting final blend was then tableted to obtain the desired tablet weight and hardness (typical according to USP General Chapters: <1216> Tablet Friability)..

An Opadry II coating suspension (18%w/w solids) was prepared to coat the tablets. The suspension was continuously stirred during the coating process. A coater (Glatt, Thomas Engineering, or Vector) was used to coat the tablets to a tablet weight gain of 2 - 3.5 %w/w, which was sufficient.

### Reference EXAMPLE 6

### Atazanavir Sulfate Tablets

A compressed tablet having a dose of 300 mg (as Free Base) was prepared having the following composition.

| Ingredient | | % (w/w) of the final composition |
|---|---|---|
| Atazanavir (as salt) | Intragranular | 48.8 |
| Stearic acid | | 2.4 |
| Microcrystalline cellulose | | 6.4 |
| Sodium starch glycolate | | 1.2 |
| Crospovidone | | 1.2 |
| HPC | | 0.6 |
| Microcrystalline cellulose | Extragranular | 33.65 |
| Sodium starch glycolate | | 3 |
| Crospovidone | | 2 |
| Magnesium stearate | | 0.75 |

| | | |
|---|---|---|
| Coating: Opadry II, based on 3% coat | | |

The preparation of atazanavir tablets was commenced by blending the intragranular ingredients in a tumbling type blender, e.g., V-blender, in a 3-step process. Firstly, a portion of atazanavir sulfate (12% of total atazanavir sulfate weight) and stearic acid was blended for 125 revolutions. Secondly, the remaining atazanavir sulfate was added and blended for another 250 revolutions. Thirdly, microcrystalline cellulose, sodium starch glycolate and crospovidone were added and the mixture was further blended for 250 revolutions.

The intragranular blend was transferred to a high shear mixer, e.g., 65L Diosna or Glatt-Fuji. A HPC solution was prepared and transferred to a foam generator (Dow Chemical Company) to make HPC foam. Foam quality (expressed as: (volume of air- volume of HPC solution)/(volume of air)X 100) was greater than 70%. HPC weight ranged from 0.5 - 3 %w/w of dry weight of intragranular blend, water to make the HPC solution ranged from 30 - 38%w/w of dry weight of intragranular blend. Granulation of the intragranular powder with HPC foam was conducted at the following mixing speeds: 90 - 200 RPM impeller speed (depending on batch size and high-shear mixer type), 1300 - 1770 RPM chopper speed. After completion of addition of calculated amount of HPC solution as foam, wet-massing was conducted without stopping the high-shear mixer. Wet massing time ranged from 0.5 - 2 min.

The wet granulation was transferred to a fluid-bed dryer and dried to a level where loss on drying was not more than 4.5%w/w.

The dried granulation was sized through a 1 mm screen.

The milled granulation was blended with calculated extragranular microcrystalline cellulose, sodium starch glycolate and crospovidone in a tumbling type blender for 250 revolutions. Magnesium stearate was then added to the blend and blended for 75 revolutions.

The resulting final blend was then tableted to obtain the desired tablet weight and hardness (typical according to USP General Chapters: <1216> Tablet Friability).

An Opadry II coating suspension (12-18%w/w solids) was prepared to coat the tablets. The suspension was continuously stirred during the coating process. A suitable coater was used to coat the tablets to a tablet weight gain of 2 - 3.5 %w/w, which was sufficient.

### Reference EXAMPLE 7

### Atazanavir Sulfate Tablets

A compressed tablet having a dose of 300 mg (as Free Base) was prepared having the following composition.

| Ingredient | | % (w/w) of the final composition |
|---|---|---|
| Atazanavir (as salt) | Intragranular | 68.3 |
| Stearic Acid | | 3.4 |
| Microcrystalline cellulose | | 8.7 |
| Sodium starch glycolate | | 1.7 |
| Crospovidone | | 2.5 |
| Povidone | | 0.2 |
| Microcrystalline cellulose | Extragranular | 11.2 |
| Crospovidone | | 3.0 |
| Magnesium Stearate | | 1.0 |

| | | |
|---|---|---|
| Coating: Opadry II, based on 3% coat | | |

The preparation of atazanavir tablets was commenced by blending the intragranular ingredients in a tumbling type blender, e.g., V-blender, in a 3-step process. Firstly, a portion of atazanavir sulfate (12% of total atazanavir sulfate weight) and stearic acid was blended for 125 revolutions. Secondly, the remaining atazanavir sulfate was added and blended for another 250 revolutions. Thirdly, microcrystalline cellulose, sodium starch glycolate, crospovidone and povidone were added and the mixture was further blended for 250 revolutions.

The intragranular blend was transferred to a high shear mixer, e.g., 65L Diosona or Glatt-Fuji. Granulation of the intragranular powder with water was conducted at the following mixing speeds: 90 - 200 RPM impeller speed (depending on batch size and high-shear mixer type), 1300 - 1770 RPM chopper speed. After completion of addition of calculated amount of water, wet-massing was conducted without stopping the high-shear mixer. Wet massing time ranged from 0.5 - 2 min.

The wet granulation was transferred to a fluid-bed dryer and dried to a level where loss on drying was not more than 4.5%w/w.

The dried granulation was sized through a 1 mm screen.

The milled granulation was blended with calculated extragranular microcrystalline cellulose and crospovidone in a tumbling type blender, e.g., V-blender, for 250 revolutions. Magnesium stearate was then added to the blend and blended for 75 revolutions.

The resulting final blend was then tableted to obtain the desired tablet weight and hardness (typical according to USP General Chapters: <1216> Tablet Friability).

An Opadry II coating suspension (18%w/w solids) was prepared to coat the tablets. The suspension was continuously stirred during the coating process. A suitable coater was used to coat the tablets to a tablet weight gain of 2 - 3.5 %w/w, which was sufficient.

### Reference EXAMPLE 8

### Atazanavir Sulfate Tablets

A compressed tablet having a dose of 300 mg (as Free Base) was prepared having the following composition.

| Ingredient | | % (w/w) of the final composition |
|---|---|---|
| Atazanavir (as salt) | Intragranular | 56.9 |
| Silicon dioxide | | 1.8 |
| Microcrystalline cellulose | | 8.8 |
| Sodium starch glycolate | | 1.4 |
| Crospovidone | | 1.4 |
| HPC | | 0.4 |
| Microcrystalline cellulose | Extragranular | 23.3 |
| Sodium starch glycolate | | 3 |
| Crospovidone | | 2 |
| Magnesium stearate | | 1 |

The preparation of atazanavir tablets was commenced by blending the intragranular ingredients. Firstly, a portion of atazanavir sulfate (34% of total atazanavir sulfate weight) and silicon dioxide was blended in a tumbling type blender, e.g., V-blender, for 2 minutes. The mixture was transferred to a high shear mixer, e.g., 65L Diosona or Glatt-Fuji, and the remaining amount of API was added, and blended for 1 mien (impeller blade at 600 RPM, chopper at 1300 RPM). Microcrystalline cellulose, sodium starch glycolate, crospovidone and HPC was added, and further blended for 2 minutes (impeller blade at 600 RPM, chopper at 1300 RPM).

Granulation of the intragranular powder with water was conducted at the following mixing speeds: 400 RPM impeller speed, 1300 RPM chopper speed. After completion of addition water, wet-massing was conducted for 2.5 minutes without stopping the high-shear mixer.

The wet granulation was dried to a level where loss on drying was not more than 3%w/w.

The dried granulation was sized through a 1 mm screen.

The milled granulation was blended with calculated extragranular microcrystalline cellulose, sodium starch glycolate and crospovidone in a tumbling type blender for 420 revolutions. Magnesium stearate was then added to the blend and blended for 126 revolutions.

The resulting final blend was then tableted to obtain the desired tablet weight and hardness (typical according to USP General Chapters: <1216> Tablet Friability).

### COMPARATIVE EXAMPLE 9

### Atazanavir Sulfate Tablets

A compressed tablet having a dose of 300 mg (as Free Base) was prepared having the following composition.

| Ingredient | | % (w/w) of the final composition |
|---|---|---|
| Atazanavir (as salt) | Intragranular | 56.9 |
| Microcrystalline cellulose | | 10.6 |
| Sodium starch glycolate | | 1.4 |
| Crospovidone | | 1.4 |
| HPC | | 0.4 |
| Microcrystalline cellulose | Extragranular | 23.3 |
| Sodium starch glycolate | | 3 |
| Crospovidone | | 2 |
| Magnesium stearate | | 1 |

The preparation of atazanavir tablets was commenced by blending the intragranular ingredients. The intragranular ingredients were added to a high shear mixer, e.g., 65L Diosona or Glatt-Fuji, in the order as in the table and mixed for 2 minutes (impeller blade at 600 RPM, chopper at 1200 RPM).

Granulation of the intragranular powder with water was conducted at the following mixing speeds: 300 RPM impeller speed, 1200 RPM chopper speed. After completion of addition water, wet-massing was conducted for 0.5 minutes without stopping the high-shear mixer.

The wet granulation was dried to a level where loss on drying was not more than 3%w/w.

The dried granulation was sized through a 1 mm screen.

The milled granulation was blended with calculated extragranular microcrystalline cellulose, sodium starch glycolate and crospovidone in a tumbling type blender, e.g., V-blender, for 420 revolutions. Magnesium stearate was then added to the blend and blended for 126 revolutions.

The resulting final blend was then tableted to obtain the desired tablet weight and hardness (typical according to USP General Chapters: <1216> Tablet Friability).

### Reference EXAMPLE 10

### Dissolution Properties

The compressed tablets of Examples 4 and 9 were tested for dissolution properties. The dissolution media was 50 millimolar ("mM") citrate buffer, pH 2.8, 1000 ml; dissolution conditions are 50 RPM paddle speed, 37°C.

The dissolution is expressed as % label claim dissolved, which is a common term used in the art to define the percentage of the dose, e.g., 300mg, that has dissolved in a given time, e.g., 60 min.

| Time, min. | Comparative Example 9 % label claim dissolved | Example 1 % label claim dissolved |
|---|---|---|
| 0 | 0 | 0 |
| 5 | 64 | 59 |
| 10 | 78 | 76 |
| 15 | 84 | 83 |
| 20 | 87 | 88 |
| 30 | 90 | 93 |
| 45 | 91 | 96 |
| 60 | 91 | 97 |

Quite surprisingly, it was found that by incorporating a lubricant intergranularly that enhanced dissolution properties were observed as the dissolution time progressed. For example, at dissolution times of up to about 20 minutes, the dissolution rate was essentially equivalent, whereas at longer dissolution times, e.g., 45 and 60 minutes, the dissolution rate of the compressed tablet in accordance with the invention was significantly higher, e.g., 6.6% higher at 60 minutes. Furthermore, it was found that the preparation of the granules in accordance with the present invention was substantially more efficient when the intragranular lubricant was included. Significantly less sticking of the material on the shearing equipment was observed.

### Reference EXAMPLE 11

### Atazanavir Sulfate Combination Tablets

A compressed bilayer tablet having a dose of atazanavir (atazanavir sulfate) 300 mg (as Free Base) in one layer with emtricitabine/tenofovir DF (200 mg/300mg) in another layer was prepared having the following composition.

| Ingredient | % (w/w) of the atazanavir layer | % (w/w) of the final composition |
|---|---|---|
| Atazanavir (as salt) | 56.9 | 27.3 |
| Stearic acid | 2.8 | 1.3 |
| Microcrystalline cellulose | 31.05 | 14.9 |
| Sodium starch glycolate | 4.4 | 2.1 |
| Crospovidone | 3.4 | 1.6 |
| HPC | 0.7 | 0.3 |
| Magnesium stearate | 0.75 | 0.4 |
| Emtricitabine/tenofovir DF formulation | - | 52.1 |

A procedure substantially as described in Example 4 was followed to make the tablet. The atazanavir sulfate formulation was tableted as a layer in a bi-layer tablet with emtricitabine/tenofovir DF in another layer to obtain the desired tablet weight and hardness (typical according to USP General Chapters: <1216> Tablet Friability).

A compressed monolith tablet having a dose of atazanavir (atazanavir sulfate) 300 mg (as Free Base) with emtricitabine/tenofovir DF (200 mg/300mg) was prepared having the above composition. A procedure substantially as described in Example 4 was followed to make the tablet where the emtricitabine/tenofovir DF was combined with the atazanavir in the initial blending step.

The atazanavir sulfate/ emtricitabine/tenofovir DF formulation was tableted as a single layer tablet to obtain the desired tablet weight and hardness hardness (typical according to USP General Chapters: <1216> Tablet Friability).

In addition, although the granules of the invention are described in the context of compressed tablets, other delivery forms are possible. Pharmaceutical compositions for oral administration can be obtained by combining the active ingredient, e.g., atazanavir sulfate, with solid carriers, granulating a resulting mixture, and processing the mixture, if desired or necessary, after the addition of appropriate excipients, into tablets, dragée cores, capsules or powders for oral use.

## Claims

1. A compressed tablet comprising abacavir sulfate and granules containing atazanavir sulfate and an intragranular lubricant, said granules having an interior section and an exterior surface and wherein at least a portion of the intragranular lubricant is present in the interior section of the granules.

2. A compressed tablet according to claim 1 comprising from 0.1 to 10% of the intragranular lubricant based on the total weight of the compressed tablet.

3. A compressed tablet according to claim 1 comprising from 0.5 to 8% of the intragranular lubricant based on the total weight of the compressed tablet.

4. A compressed tablet according to any one of the preceding claims wherein the intragranular lubricant is selected from the group consisting of magnesium stearate, zinc stearate, calcium stearate, stearic acid, palmitic acid, sodium stearyl fumarate, sodium benzoate, sodium lauryl sulfate, glyceryl monostearate glyceryl palmitostearate, hydrogenated castor oil, hydrogenated vegetable oil, mineral oil, carnauba wax, polyethylene glycol and mixtures thereof.

5. A compressed tablet according to any one of the preceding claims further comprising from 1 to 20%, based on the total weight of the compressed tablet, of a disintegrant.

6. A compressed tablet according to any one of the preceding claims further comprising from 0.1 to 10%, based on the total weight of the compressed tablet, of a binder.

7. A compressed tablet according to any one of the preceding claims further comprising from 5 to 90%, based on the total weight of the compressed tablet, of a filler.

8. A compressed tablet according to any one of the preceding claims further comprising from 0.1 to 3%, based on the total weight of the compressed tablet, of an extragranular lubricant.

9. A compressed tablet according to any one of the preceding claims further comprising lamivudine.

10. A compressed table according to claim 9 comprising abacavir sulfate, lamivudine, atazanavir sulfate, an intragranular lubricant, and an extragranular lubricant wherein said tablet is prepared via wet granulation in which the atazanavir sulfate and the intragranular lubricant are blended intragranularly and the extragranular lubricant is added extragranuarly.

11. A compressed tablet according to claim 10 in the form of a multi-layer tablet wherein the atazanavir sulfate is in one layer and at least one of the abacavir sulfate or lamivudine is in another layer.

12. A compressed tablet according to claim 9 in the form of a monolith tablet wherein the atazanavir sulphate, abacavir sulfate and lamivudine and the other agent are in the same layer.

13. A compressed tablet according to claim 9 wherein said tablet is prepared via wet granulation in which (i) at least one of the abacavir sulfate or lamivudine, (ii) the atazanavir sulfate and (iii) the intragranular lubricant are blended intragranularly.

14. A compressed tablet according to claim 9 wherein said tablet is prepared via wet granulation in which the atazanavir sulfate and the intragranular lubricant are blended intragranularly and at least one of the abacavir sulfate or lamivudine is added extragranuarly.

15. A compressed tablet according to any one of the preceding claims for use in a method of treating an HIV infection.

## Patentansprüche

1. Komprimierte Tablette, umfassend Abacavirsulfat und Granulat mit Atazanavirsulfat und ein intragranuläres Gleitmittel, wobei das Granulat einen Innenteil und eine äußere Oberfläche aufweist und worin mindestens ein Teil des intragranulären Gleitmittels im Innenteil des Granulats vorliegt.

2. Komprimierte Tablette nach Anspruch 1, umfassend von 0,1 bis 10 % des intragranulären Gleitmittels, bezogen auf das Gesamtgewicht der komprimierten Tablette.

3. Komprimierte Tablette nach Anspruch 1, umfassend von 0,5 bis 8 % des intragranulären Gleitmittels, bezogen auf das Gesamtgewicht der komprimierten Tablette.

4. Komprimierte Tablette nach einem der vorangehenden Ansprüche, worin das intragranuläre Gleitmittel ausgewählt ist aus Magnesiumstearat, Zinkstearat, Calciumstearat, Stearinsäure, Palmitinsäure, Natriumstearylfumarat, Natriumbenzoat, Natriumlaurylsulfat, Glycerylmonostearat, Glycerylpalmitostearat, hydriertes Ricinusöl, hydriertes Pflanzenöl, Mineralöl, Carnaubawachs, Polyethylenglykol und Gemischen davon.

5. Komprimierte Tablette nach einem der vorangehenden Ansprüche, ferner umfassend von 1 bis 20 % eines Zerfallsmittels, bezogen auf das Gesamtgewicht der komprimierten Tablette.

6. Komprimierte Tablette nach einem der vorangehenden Ansprüche, ferner umfassend von 0,1 bis 10 % eines Bindemittels, bezogen auf das Gesamtgewicht der komprimierten Tablette.

7. Komprimierte Tablette nach einem der vorangehenden Ansprüche, ferner umfassend von 5 bis 90 % eines Füllmittels, bezogen auf das Gesamtgewicht der komprimierten Tablette.

8. Komprimierte Tablette nach einem der vorangehenden Ansprüche, ferner umfassend von 0,1 bis 3 % eines extragranulären Gleitmittels, bezogen auf das Gesamtgewicht der komprimierten Tablette.

9. Komprimierte Tablette nach einem der vorangehenden Ansprüche, ferner umfassend Lamivudin.

10. Komprimierte Tablette nach Anspruch 9, umfassend Abacavirsulfat, Lamivudin, Atazanavirsulfat, ein intragranuläres Gleitmittel und ein extragranuläres Gleitmittel, worin die Tablette über Feuchtgranulierung hergestellt wird, worin das Atazanavirsulfat und das intragranuläre Gleitmittel intragranulär gemischt werden und das extragranuläre Gleitmittel extragranulär zugefügt wird.

11. Komprimierte Tablette nach Anspruch 10 in der Form einer Mehrschichttablette, worin das Atazanavirsulfat in einer Schicht ist und mindestens eines vom Abacavirsulfat oder Lamivudin in einer anderen Schicht ist.

12. Komprimierte Tablette nach Anspruch 9 in der Form einer monolithischen Tablette, worin das Atazanavirsulfat, Abacavirsulfat und Lamivudin und das andere Mittel in der gleichen Schicht sind.

13. Komprimierte Tablette nach Anspruch 9, worin die Tablette über Feuchtgranulierung hergestellt wird, worin (i) mindestens eines vom Abacavirsulfat oder Lamivudin, (ii) das Atazanavirsulfat und (iii) das intragranuläre Gleitmittel intragranulär gemischt werden.

14. Komprimierte Tablette nach Anspruch 9, worin die Tablette über Feuchtgranulierung hergestellt wird, worin das Atazanavirsulfat und das intragranuläre Gleitmittel intragranulär gemischt werden und mindestens eines vom Abacavirsulfat oder Lamivudin extragranulär zugefügt wird.

15. Komprimierte Tablette nach einem der vorangehenden Ansprüche zur Verwendung in einem Verfahren zur Behandlung einer HIV-Infektion.

## Revendications

1. Cachet comprimé comprenant du sulfate d'abacavir et des granulés contenant du sulfate d'atazanavir et un lubrifiant intragranulaire, lesdits granulés possédant une section intérieure et une surface extérieure et dans lequel au moins une portion du lubrifiant intragranulaire est présente dans la section intérieure des granulés.

2. Cachet comprimé selon la revendication 1, comprenant de 0,1 à 10% du lubrifiant intragranulaire sur la base du poids total du cachet comprimé.

3. Cachet comprimé selon la revendication 1, comprenant de 0,5 à 8% du lubrifiant intragranulaire sur la base du poids total du cachet comprimé.

4. Cachet comprimé selon l'une quelconque des revendications précédentes, dans lequel le lubrifiant intragranulaire est choisi dans le groupe constitué de stéarate de magnésium, de stéarate de zinc, de stéarate de calcium, d'acide stéarique, d'acide palmitique, de stéarylfumarate de sodium, de benzoate de sodium, de laurylsulfate de sodium, de monostéarate de glycéryle, de palmitostéarate de glycéryle, d'huile de ricin hydrogénée, d'huile végétale hydrogénée, d'huile minérale, de cire de carnauba, de polyéthylèneglycol et de leurs mélanges.

5. Cachet comprimé selon l'une quelconque des revendications précédentes, comprenant en outre de 1 à 20%, sur la base du poids total du cachet comprimé, d'un désintégrant.

6. Cachet comprimé selon l'une quelconque des revendications précédentes, comprenant en outre de 0,1 à 10%, sur la base du poids total du cachet comprimé, d'un liant.

7. Cachet comprimé selon l'une quelconque des revendications précédentes, comprenant en outre de 5 à 90%, sur la base du poids total du cachet comprimé, d'une charge.

8. Cachet comprimé selon l'une quelconque des revendications précédentes, comprenant en outre de 0,1 à 3%, sur la base du poids total du cachet comprimé, d'un lubrifiant extragranulaire.

9. Cachet comprimé selon l'une quelconque des revendications précédentes, comprenant en outre de la lamivudine.

10. Cachet comprimé selon la revendication 9, comprenant du sulfate d'abacavir, de la lamivudine, du sulfate d'atazanavir, un lubrifiant intragranulaire et un lubrifiant extragranulaire, où ledit cachet est préparé via une granulation par voie humide dans laquelle le sulfate d'atazanavir et le lubrifiant intragranulaire sont mélangés de façon intragranulaire et le lubrifiant extragranulaire est ajouté de façon extragranulaire.

11. Cachet comprimé selon la revendication 10, sous la forme d'un cachet à couches multiples, dans lequel le sulfate d'atazanavir est dans une couche et au moins un du sulfate d'abacavir ou de la lamivudine est dans une autre couche.

12. Cachet comprimé selon la revendication 9, sous la forme d'un cachet monolithe, dans lequel le sulfate d'atazanavir, le sulfate d'abacavir et la lamivudine et l'autre agent sont dans la même couche.

13. Cachet comprimé selon la revendication 9, où ledit cachet est préparé via une granulation par voie humide dans laquelle (i) au moins un du sulfate d'abacavir ou de la lamivudine, (ii) le sulfate d'atazanavir et (iii) le lubrifiant intragranulaire sont mélangés de façon intragranulaire.

14. Cachet comprimé selon la revendication 9, où ledit cachet est préparé via une granulation par voie humide dans laquelle le sulfate d'atazanavir et le lubrifiant intragranulaire sont mélangés de façon intragranulaire et au moins un du sulfate d'abacavir ou de la lamivudine est ajouté de façon extragranulaire.

15. Cachet comprimé selon l'une quelconque des revendications précédentes, pour une utilisation dans une méthode de traitement d'une infection par VIH.
